(19) Europäisches Patentamt
European Patent Office
Office européen des brevets

(11) Veröffentlichungsnummer: **0 322 761 B1**

(12) **EUROPÄISCHE PATENTSCHRIFT**

(45) Veröffentlichungstag der Patentschrift: **04.03.92**

(51) Int. Cl.[5]: **C07C 209/48**, C07C 211/10, B01J 23/74

(21) Anmeldenummer: **88121485.2**

(22) Anmeldetag: **22.12.88**

Die Akte enthält technische Angaben, die nach dem Eingang der Anmeldung eingereicht wurden und die nicht in dieser Patentschrift enthalten sind.

# (54) Verfahren zur Herstellung von Dialkylaminoethylaminen.

(30) Priorität: **30.12.87 DE 3744506**

(43) Veröffentlichungstag der Anmeldung:
**05.07.89 Patentblatt 89/27**

(45) Bekanntmachung des Hinweises auf die Patenterteilung:
**04.03.92 Patentblatt 92/10**

(84) Benannte Vertragsstaaten:
**AT BE CH DE ES FR GB IT LI NL SE**

(56) Entgegenhaltungen:
**EP-A- 0 013 275**
**EP-A- 0 037 716**
**EP-A- 0 151 986**
**EP-A- 0 168 096**
**DE-A- 1 543 337**
**DE-A- 1 667 106**
**GB-A- 745 684**
**US-A- 2 436 368**
**US-A- 4 140 720**

(73) Patentinhaber: **HOECHST AKTIENGESELL-SCHAFT**
**Postfach 80 03 20**
**W-6230 Frankfurt am Main 80(DE)**

(72) Erfinder: **Weber, Jürgen, Dr. Dipl.-Chem.**
**Bunsenstrasse 17**
**W-4200 Oberhausen 11(DE)**
Erfinder: **Falk, Volker, Dr. Dipl.-Chem.**
**Am Dausendbusch 17**
**W-5600 Wuppertal 2(DE)**
Erfinder: **Horn, Gerhardt, Dr. Dipl.-Chem.**
**Bunsenstrasse 19**
**W-4200 Oberhausen 11(DE)**
Erfinder: **Bach, Hanswilhelm, Dr. Dipl.-Chem.**
**Alleestrasse 56**
**W-4100 Duisburg 11(DE)**
Erfinder: **Mathieu, Klaus**
**Dinslakener Strasse 123**
**W-4200 Oberhausen 11(DE)**
Erfinder: **Kniep, Claus**
**Rosenstrasse 93**
**W-4200 Oberhausen 1(DE)**

**EP 0 322 761 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Dialkylaminoethylaminen durch Hydrieren von Dialkylaminoacetonitrilen an Kobaltkatalysatoren.

Dialkylaminoethylamine sind gefragte Zwischenprodukte in der chemischen Industrie für die Herstellung einer Reihe von Arzneimitteln. So ist die Diethylaminoethylamin-Verbindung ein wichtiges Ausgangsmaterial für die Synthesen von Procainamid, Ambenoniumchlorid, Metoclopramid und Chinchocain.

Dialkylaminoethylamine sind auf mehreren Wegen zugänglich. Ein in verschiedenen Varianten in der Literatur beschriebenes Verfahren ist die Umsetzung von Kalium-Phthalsäureimid mit Dialkylaminoethylhalogeniden. Alternativ kann die Reaktion auch mit Ammoniak oder Hexamethylentetramin statt Kalium-Phthalsäureimid durchgeführt werden. Nachteile dieses Prozesse sind der hohe Chemikalienaufwand und die durch die Umsetzungsprodukte verursachte Belastung der Abwässer. Einer Anwendung dieser Verfahren im technischen Maßstab sind daher Grenzen gesetzt.

Sehr kostengünstig ist die Umsetzung von Ethylenimin mit Dialkylaminen. Sie ist jedoch an das Vorhandensein des hochtoxischen Ethylenimins gebunden.

Ein weiterer Weg, der zu Dialkylaminoethylaminen führt, ist die Mannich-Reaktion von Dialkylaminen, Formaldehyd und Alkalicyanid, die Dialkylaminoacetonitrile ergibt. Durch Hydrieren erhält man aus den Acetonitrilen schließlich das gewünschte Diamin.

Die Umwandlung der Nitrilgruppe in die Aminogruppe kann mit Natrium oder Lithiumaluminiumhydrid erfolgen. Industrielle Bedeutung besitzen diese Verfahren nicht. In der Technik führt man die Hydrierung mit Wasserstoff in Gegenwart von Katalysatoren durch.

So läßt sich Diethylaminoacetonitril nach Winans und Adkins (Am.Soc. 55, 4167 (1933)) an Raney-Nickel mit 37 %iger Ausbeute zum Diamin hydrieren. Abgesehen davon, daß eine solche Ausbeute für ein technisches Verfahren zu gering ist, konnten die Angaben von Winans und Adkins nicht ohne weiteres reproduziert werden (vgl. Houben-Weyl 11/1, Seite 563).

In der GB-PS 745 684 ist ein Verfahren zur Herstellung von N,N-Dialkylaminoethylamin durch katalytisches Hydrieren von N,N-Dialkylaminoacetonitril bei überatmosphärischem Druck und bei Temperaturen unterhalb 110 °C beschrieben. Dieser Prozeß erbringt eine Ausbeute von 92 %, erfordert aber den Einsatz von flüssigem Ammoniak und von Raney-Kobalt, dessen Herstellung aufwendig ist. Überdies können Raney-Katalysatoren nicht als Festbett angeordnet, sondern nur in Suspension eingesetzt werden.

Es bestand daher die Aufgabe, ein Verfahren zu entwickeln, das die geschilderten Nachteile des Standes der Technik überwindet und die Hydrierung von Dialkylaminoacetonitrilen mit vertretbaren technischen Mitteln in hoher Ausbeute ermöglicht.

Die Erfindung besteht in einem Verfahren zur Herstellung von Dialkylaminoethylaminen durch Hydrierung von Dialkylaminoacetonitrilen in Gegenwart von Kobaltkatalysatoren. Es ist dadurch gekennzeichnet, daß die Hydrierung bei 40 bis 120 °C und bei 4 bis 15 MPa an Katalysatoren erfolgt, die durch Ausfällen von Kobaltcarbonat aus der wäßrigen Lösung eines Kobaltsalzes mit einer wäßrigen Alkalicarbonatlösung bei 20 bis 95 °C, Filtrieren, Auswaschen, gegebenenfalls Formen der Katalysatormasse und anschließende Reduktion mit Wasserstoff bei Temperaturen zwischen 200 und 300 °C, vorzugsweise 220 und 280 °C erhalten wurden.

Überraschenderweise ermöglicht das neue Verfahren, Dialkylaminoacetonitrile in einfacher Weise mit hoher Ausbeute und hoher Selektivität in Dialkylaminoethylamine zu überführen.

Ein wesentliches Merkmal der Erfindung ist der Einsatz auf bestimmtem Wege hergestellter Kobaltkatalysatoren für die Hydrierung der Nitrile.

Diese Katalysatoren bestehen im reduzierten Zustand aus Kobalt. Nach einer bevorzugten Ausführungsform enthalten sie außerdem 0,25 bis 15 Gew.-% $SiO_2$, $MnO_2$, $ZrO_2$, $Al_2O_3$ oder MgO in Form der Oxide, Hydroxide oder Oxidhydrate. Diese Zusatzstoffe werden einzeln verwendet oder als Kombination von zwei oder mehreren der Substanzen. Bevorzugt beträgt ihr Anteil 1 bis 8 und insbesondere 2 bis 5 Gew.-%. Alle vorstehenden Angaben in Gewichtsprozent beziehen sich auf die gesamte Katalysatormasse im wasserfreien Zustand vor der Reduktion.

Die oben aufgeführten Formeln dienen lediglich der Beschreibung der quantitativen Zusammensetzung der Katalysatormasse, sie geben nicht unbedingt die genaue chemische Struktur der Zusatzstoffe wieder. Außer als Oxide können sie im nichtreduzierten wie auch im reduzierten Katalysator als Hydroxide und insbesondere als Oxidhydrate enthalten sein.

Die Wirkungsweise der Zusatzstoffe ist nicht in allen Einzelheiten geklärt. Versuchsergebnisse sprechen dafür, daß sie die Struktur des Katalysators, insbesondere seine Oberflächenstruktur gegenüber Sinterungseffekten bei hohen Temperaturen stabilisieren. Darüber hinaus erhöhen sie auch die mechanische Stabilität der aus der Katalysatormasse hergestellten Formkörper.

Zur Herstellung der Kobaltkatalysatoren fällt man bei 20 bis 95 °C Kobaltcarbonat aus der wäßrigen Lösung eines Kobaltsalzes mit einer wäßrigen Alkalicarbonatlösung. Unter dem Begriff Kobaltcarbonat wird vereinfacht das Umsetzungsprodukt aus Kobaltsalz und Alkalicarbonat unter den gewählten Reaktionsbedingungen verstanden, das nicht der Formel $CoCO_3$ entsprechen muß, sondern z.B. auch basische Kobaltcarbonate umfassen kann. Geeignete Kobaltsalze sind z.B. Kobaltnitrat, -chlorid, -sulfat, -acetat. Als Alkalicarbonate kommen insbesondere die Natrium- oder die Kaliumverbindung in Betracht. Die Lösungen der Ausgangsstoffe enthalten Kobaltsalz bzw. Alkalicarbonat jeweils in Konzentrationen von 25 bis 150 g Co bzw. Alkalicarbonat/l Lösung. Kobaltsalz und Alkalicarbonat können in äquimolaren Mengen miteinander umgesetzt werden, zweckmäßiger ist es jedoch, mit einem Alkalicarbonatüberschuß zu arbeiten. Bewährt hat es sich, 1,1 bis 1,5 und insbesondere 1,2 bis 1,3 Mol Alkalicarbonat je Mol Kobaltsalz zu verwenden.

Zur Herstellung einen oder mehrere Zusatzstoffe enthaltender Kobaltkatalysatoren kann man die entsprechenden Substanzen in der Lösung des Kobaltsalzes oder in der Alkalicarbonatlösung suspendieren. Mit gleich gutem Erfolg kann man aber auch ein lösliches Salz des Zusatzstoffes vor der Fällung zur Kobaltsalzlösung geben und Kobaltcarbonat und Zusatzstoff gemeinsam ausfällen. Schließlich ist es auch möglich, die Kobaltcarbonatfällung getrennt vorzunehmen und anschließend den Zusatzstoff auf das Kobaltcarbonat aufzufällen.

Von großer Bedeutung für die Leistungsfähigkeit des Katalysators ist die Durchführung seiner Reduktion und damit seiner Aktivierung. Sie erfolgt in einem Temperaturbereich, der bei 200 °C, vorzugsweise 220 °C beginnt und 300 °C, bevorzugt 280 °C nicht übersteigt. Besonders zweckmäßig ist es, in mindestens drei Stufen bei von Stufe zu Stufe ansteigender Temperatur zu reduzieren.

Als Reduktionsmittel verwendet man Wasserstoff. Er wird mit einer Raumgeschwindigkeit von 200 bis 2000 l $H_2$ je 1 Katalysator und Stunde (200 bis 2000 $V_H/V_{Kat}$ . h), bevorzugt 300 bis 1000 $V_H/V_{Kat}$ . h und insbesondere 400 bis 700 $V_H/V_{Kat}$ . h bei Normaldruck über die Katalysatorschüttung geleitet.

Es hat sich bewährt, in der ersten Stufe eine Temperatur von 220 bis 250 °C, vorzugsweise 230 bis 240 °C einzustellen und 1 bis 4, insbesondere 2 bis 3 Stunden aufrechtzuerhalten. In der zweiten Stufe erfolgt die Reduktion während 1 bis 5, insbesondere 2 bis 3 Stunden bei 245 bis 260 °C, vorzugsweise 250 bis 255 °C. Anschließend wird die Reduktion weitere 1 bis 5, insbesondere 2 bis 3 Stunden bei 255 bis 280 °C, vorzugsweise bei 260 bis 270 °C zu Ende geführt.

Der reduzierte Katalysator ist pyrophor und an der Luft selbstentzündlich. Zur besseren Handhabung behandelt man ihn daher mit Sauerstoff, der durch ein Inertgas stark verdünnt ist. Beispielsweise läßt man einen $N_2$-Strom auf ihn einwirken, der Sauerstoff in einer Konzentration von etwa 0,5 bis etwa 1 Vol.-%, enthält. Durch diese Behandlung wird der Katalysator oberflächlich oxidiert; er ist in diesem Zustand an der Luft bis etwa 80 °C stabil und nicht selbstentzündlich.

Das neue Verfahren erlaubt es, Dialkylaminoacetonitrile sehr selektiv und in hoher Ausbeute in die entsprechenden Dialkylaminoethylamine zu überführen. Zur Hydrierung können die Nitrile in der handelsüblichen technischen Form eingesetzt werden, eine besondere Vorbehandlung, z.B. um Verunreinigungen, die von Herstellung herrühren, zu beseitigen, ist nicht erforderlich. Die Hydrierung selbst wird bei Temperaturen von 40 bis 120 °C und bei Drücken von 4 bis 15 MPa durchgeführt. Besonders bewährt hat es sich, bei 45 bis 100 °C, vorzugsweise 50 bis 80 °C und bei 6 bis 12, vorzugsweise 8 bis 10 MPa zu arbeiten.

Die Dialkylaminoacetonitrile können in Substanz eingesetzt werden. Zweckmäßig ist es jedoch, sie in einem inerten Lösungsmittel gelöst dem Hydrierreaktor zuzuführen. Als Lösungsmittel können aliphatische, cycloaliphatische und aromatische Kohlenwasserstoffe und aliphatische Alkohole Anwendung finden. Geeignete Lösungsmittel sind z.B. Cyclohexan, Toluol, Butanol, 2-Ethylhexanol. Besonders bewährt hat sich Cyclohexan. Die Konzentration der Dialkylaminoacetonitrile in der Lösung beträgt (jeweils bezogen auf die Lösung) zwischen 5 und 50 Gew.-%, vorzugsweise 10 bis 40 und insbesondere 15 bis 30 Gew.-%.

Nach einer bevorzugten Ausführungsform setzt man dem Nitril Ammoniak zu. Man erzielt dadurch eine Erhöhung der Selektivität der Hydrierung zu Dialkylaminoethylaminen auf über 90%. Es hat sich bewährt, je Mol Nitril 1 bis 20 Mol Ammoniak anzuwenden. Besonders gute Ergebnisse erzielt man, wenn je Mol Nitril 1,5 bis 15 und insbesondere 2 bis 10 Mol Ammoniak in dem Ausgangsgemisch enthalten ist.

Das neue Verfahren erlaubt es, Dialkylaminoacetonitrile der allgemeinen Formel

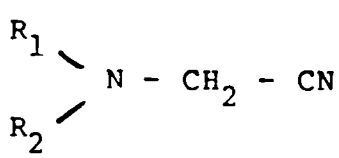

wobei $R_1$ und $R_2$ gleich oder verschieden sind und jeweils unverzweigte oder verzweigte Alkylreste mit 1 bis 9 Kohlenstoffatomen bedeuten, zu den entsprechenden Diaminen zu hydrieren. Besonders

geeignet ist es zur Hydrierung von Alkylaminoacetonitrilen, in denen $R_1$ und $R_2$ gleich sind und jeweils für unverzweigte oder verzweigte Alkylreste mit 1 bis 6 Kohlenstoffatome stehen, insbesondere für unverzweigte oder verzweigte Alkylreste mit jeweils 2 bis 4 Kohlenstoffatomen.

Die Hydrierung der Dialkylaminoacetonitrile kann sowohl diskontinuierlich als auch insbesondere kontinuierlich durchgeführt werden. Bei kontinuierlicher Durchführung ist der Katalysator in einem Rohr angeordnet, dem das Ausgangsgemisch am Boden zugeführt wird. Eine ausreichende Vorheizung der Einsatzstoffe ist vorzusehen. Es empfiehlt sich, die Raumgeschwindigkeit auf Werte zwischen 0,05 und 1,0, insbesondere 0,1 bis 0,5 Volumen Dialkylaminoacetonitril bzw. Dialkylaminoacetonitril-Lösung je Volumen Katalysator und Stunde einzustellen.

In den folgenden Beispielen wird die Herstellung der erfindungsgemäß eingesetzten Katalysatoren und die Hydrierung von Dialkylaminoacetonitrilen beschrieben. Es ist nicht beabsichtigt, die Erfindung auf diese speziellen Ausführungsformen zu beschränken.

Beispiel 1: Herstellung eines Kobaltkatalysators ohne Zusatzstoffe

In eine auf 90°C erhitzte Lösung von 800 g $Na_2CO_3$ in 7,5 l entionisiertem Wasser läßt man innerhalb von 2 min unter kräftigem Rühren eine auf 95°C erhitzte Lösung von 1852 g $Co(NO_3)_2 \cdot 6H_2O$ (≙ 375 g Co) in 7,5 l entionisiertem Wasser einfließen. Es entsteht eine Suspension von Kobaltcarbonat in Wasser mit einem pH-Wert von 8,2 bis 8,4. Das Fällungsprodukt wird abfiltriert und mit etwa 90 l Kondensat-Wasser (Temperatur: 70°C) intensiv gewaschen, so daß die Leitfähigkeit des Waschwassers nach Beendigung des Waschvorganges kleiner als 100 µS ist. Das noch feuchte Katalysatorvorprodukt wird erneut in entionisiertem Wasser suspendiert und darauf sprühgetrocknet. Die Masse enthält etwa 53,5 Gew.-% Kobalt.

Zur Reduktion werden in einem Rohrreaktor (Durchmesser: 50 mm) über 0,5 l der getrockneten Katalysatormasse bei 240°C während 2 h 200 l $H_2$/h geleitet. Darauf erhöht man die Temperatur auf 250°C und reduziert weitere 2 h mit 200 l $H_2$/h und führt die Reduktion durch Behandlung des Katalysators während weiterer 2 h mit 200 l $H_2$/h bei 260°C zuende. Zur Stabilisierung behandelt man das Pulver bei 50 bis 70°C mit einem 0,7 Vol.-% $O_2$ enthaltenden $N_2$-Strom und preßt es anschließend zu Tabletten.

Beispiel 2: Herstellung eines Kobaltkatalysators mit Zusatzstoff.

In eine auf 90°C erhitzte Lösung von 840 g $Na_2CO_3$ in 7,5 l entionisiertem Wasser läßt man innerhalb von 2 min unter kräftigem Rühren eine auf 95°C erhitzte Lösung von 1852 g $Co(NO_3)_2 \cdot 6H_2O$ (≙ 375 g Co) und 85,73 g Mn $(NO_3)_2 \cdot 4H_2O$ in 7,5 l entionisiertem Wasser gleichmäßig einfließen. Es entsteht eine Suspension von Kobalt- und Mangancarbonat in Wasser mit einem pH-Wert von 8,2 bis 8,4. Das Fällungsprodukt wird abfiltriert und mit etwa 90 l 70°C heißem Kondensat-Wasser intensiv gewaschen, so daß die Leitfähigkeit des Waschwassers nach Beendigung des Waschvorganges kleiner als 100 µS ist.

Das noch feuchte Katalysatorvorprodukt wird erneut in entionisiertem Wasser suspendiert und darauf sprühgetrocknet. Die Masse enthält etwa 52 Gew.-% Co und etwa 4,1 Gew.-% $MnO_2$.

Die Reduktion des Katalysators erfolgt wie in Beispiel 1 beschrieben.

Beispiel 3: Hydrierung von Diethylaminoacetonitril

In einem beheizbaren Doppelmantelrohr von 28 mm Innendurchmesser und 3 m Länge werden 1,8 l des in Beispiel 1 beschriebenen Kobalt-Katalysators in Form von Tabletten mit 6 mm Durchmesser als Festbett angeordnet. Man erhitzt auf 70°C und führt Wasserstoff unter einem Druck von 8 MPa sowie über eine Kolbenpumpe je Stunde kontinuierlich 600 ml einer Lösung von Diethylaminoactonitril in Cyclohexan (15 Gew.-% Nitril, bezogen auf die Lösung) am Boden des Reaktionsrohres zu. Das am Reaktorkopf austretende Produkt enthält kein Diethylaminoacetonitril. Gaschromatografisch werden neben 86,5 % Lösungsmittel 11 % Diethylaminoethylamin nachgewiesen.

Beispiel 4: Hydrierung von Diethylaminoacetonitril

Im Reaktor des Beispiels 3 wird unter Verwendung von 1,8 l des Katalysators aus Beispiel 2 bei 60°C und einem $H_2$-Druck von 8 MPa $H_2$ Diethylaminoacetonitril in Form einer 30 Gew.-%igen Lösung (bezogen auf die Lösung) in Cyclohexan umgesetzt. Gleichzeitig leitet man dem Reaktor 2,5 Mol $NH_3$/Mol Nitril zu und erhöht den Durchsatz auf 900 ml/h. Das Diethylaminoacetonitril ist vollständig umgesetzt. Das Reaktionsprodukt enthält nach gaschromatografischer Auswertung 73,2 % Cyclohexan und 25,1 % des Diethylaminoethylamins.

Beispiel 5: Hydrierung von Diethylaminoacetonitril

Im Reaktor des Beispiels 3 wird bei 50°C und einem $H_2$-Druck von 8 MPa unverdünntes Diethylaminoacetonitril umgesetzt. Je Mol Nitril führt man dem Reaktor 2,5 Mol $NH_3$ zu, der Durchsatz wird

auf 180 ml/h ≙ V/Vh = 0,1 eingestellt. Das Nitril wird vollständig umsetzt, das Reaktionsprodukt enthält nach gaschromatographischer Analyse 89,7 % Diethylaminoethylamin, der Rest sind Spaltprodukte. Die Aufarbeitung des Reaktionsproduktes erfolgt in einer Kolonne mit 24 theoretischen Böden. Diethylaminoethylamin wird in mehr als 99 %iger Reinheit gewonnen.

**Patentansprüche**

1. Verfahren zur Herstellung von Dialkylaminoethylaminen durch Hydrierung von Dialkylaminoacetonitrilen in Gegenwart von Kobaltkatalysatoren, dadurch gekennzeichnet, daß die Hydrierung bei 40 bis 120 °C und bei 4 bis 15 MPa an Katalysatoren erfolgt, die durch Ausfällen von Kobaltcarbonat aus der wäßrigen Lösung eines Kobaltsalzes mit einer wäßrigen Alkalicarbonatlösung bei 20 bis 95 °C, Filtrieren, Auswaschen, gegebenenfalls Formen der Katalysatormasse und anschließende Reduktion mit Wasserstoff bei Temperaturen zwischen 200 und 300 °C, vorzugsweise 220 und 280 °C erhalten wurden.

2. Verfahren nach Anspruch 1, dadurch gekennzeichnet, daß der Katalysator zusätzlich 0,25 bis 15 Gew.-%, bezogen auf die gesamte Katalysatormasse, $SiO_2$, $MnO_2$, $ZrO_2$, $Al_2O_3$ oder MgO einzeln oder als Kombination von zwei oder mehreren dieser Substanzen in Form der Oxide, Hydroxide oder Oxidhydrate enthält.

3. Verfahren nach Anspruch 2, dadurch gekennzeichnet, daß $SiO_2$, $MnO_2$, $ZrO_2$, $Al_2O_3$ oder MgO in einer Menge von 1 bis 8 und insbesondere 2 bis 5 Gew.-%, bezogen auf die gesamte Katalysatormasse, im Katalysator enthalten sind.

4. Verfahren nach einem oder mehreren der Ansprüche 1 bis 3, dadurch gekennzeichnet, daß die Reduktion in mindestens drei Stufen bei von Stufe zu Stufe ansteigender Temperatur erfolgt.

5. Verfahren nach Anspruch 4, dadurch gekennzeichnet, daß die Reduktion in der ersten Stufe bei einer Temperatur von 220 bis 250 °C, vorzugsweise 230 bis 240 °C, in der zweiten Stufe bei 245 bis 260 °C, vorzugsweise 250 bis 255 °C und in der dritten Stufe bei 255 bis 280 °C, vorzugsweise 260 bis 270 °C durchgeführt wird.

6. Verfahren nach einem oder mehreren der Ansprüche 1 bis 5, dadurch gekennzeichnet, daß die Hydrierung bei 45 bis 100 °C, vorzugsweise 50 bis 80 °C erfolgt.

7. Verfahren nach einem oder mehreren der Ansprüche 1 bis 6, dadurch gekennzeichnet, daß die Hydrierung bei 6 bis 12, vorzugsweise 8 bis 10 MPa erfolgt.

8. Verfahren nach einem oder mehreren der Ansprüche 1 bis 7, dadurch gekennzeichnet, daß die Dialkylaminoacetonitrile in einem inerten Lösungsmittel gelöst eingesetzt werden.

9. Verfahren nach Anspruch 8, dadurch gekennzeichnet, daß die Konzentration des Dialkylaminoacetonitrils in der Lösung 5 bis 50 Gew.-%, vorzugsweise 10 bis 40 Gew.-% und insbesondere 15 bis 30 Gew.-% beträgt.

10. Verfahren nach einem oder mehreren der Ansprüche 1 bis 9, dadurch gekennzeichnet, daß die Hydrierung in Gegenwart von Ammoniak erfolgt, wobei je Mol Dialkylaminoacetonitril 1 bis 20 Mol, vorzugsweise 1,5 bis 15 Mol und insbesondere 2 bis 10 Mol Ammoniak eingesetzt werden.

**Claims**

1. A process for the preparation of dialkylaminoethylamines by the hydrogenation of dialkylaminoacetonitriles in the presence of cobalt catalysts, characterised in that hydrogenation takes place at 40 to 120 °C and at 4 to 15 MPa on catalysts which have been obtained by the precipitation of cobalt carbonate out of the aqueous solution of a cobalt salt with an aqueous alkali metal carbonate solution at 20 to 95 °C filtration, washing out, optionally shaping of the catalyst mass and subsequent reduction with hydrogen at temperatures of between 200 and 300 °C, preferably between 220 and 280 °C.

2. A process according to claim 1, characterised in that the catalyst contains in addition 0.25 to 15 % by weight, related to the total catalyst mass, of $SiO_2$, $MnO_2$, $ZrO_2$, $Al_2O_3$ or MgO singly or as a combination of two of more of these substances in the form of the oxides, hydroxides or oxide hydrates.

3. A process according to claim 2, characterised in that $SiO_2$, $MnO_2$, $ZrO_2$, $Al_2O_3$ or MgO is contained in the catalyst in an amount of 1 to 8 and in particular 2 to 5 % by weight, related to the total catalyst mass.

4. A process according to one or more of the claims 1 to 3, characterised in that reduction takes place in at least three stages at a temperature which increases from stage to stage.

5. A process according to claim 4, characterised in that reduction is performed in the first stage at a temperature of 220 to 250 °C, preferably 230 to 240 °C, in the second stage at 245 to 260 °C, preferably 250 to 255 °C and in the third stage at 255 to 260 °C, preferably 260 to 270 °C.

6. A process according to one or more of the claims 1 to 5, characterised in that hydrogenation takes place at 45 to 100 °C, preferably at 50 to 80 °C.

7. A process according to one or more of the claims 1 to 6, characterised in that hydrogenation takes place at 6 to 12, preferably 8 to 10 MPa.

8. A process according to one or more of the claims 1 to 7, characterised in that the dialkylaminoacetonitriles are used dissolved in an inert solvent.

9. A process according to claim 8, characterised in that the concentration of dialkylaminoacetonitrile in the solution is 5 to 50 % by weight, preferably 10 to 40 % by weight and in particular 15 to 30 % by weight.

10. A process according to one or more of the claims 1 to 9, characterised in that hydrogenation takes place in the presence of ammonia, 1 to 20 moles, preferably 1.5 to 15 moles and in particular 2 to 10 moles ammonia being used per mole of dialkylaminoacetonitrile.

## Revendications

1. Procédé de préparation de dialkylaminoéthylamines par hydrogénation de dialkylaminoacétonitriles en présence de catalyseurs au cobalt, caractérisé en ce que l'hydrogénation est effectuée à des températures de 40 à 120 °C et des pressions de 4 à 15 MPa sur des catalyseurs qui ont été obtenus par précipitation de carbonate de cobalt à partir de la solution aqueuse d'un sel de cobalt à l'aide d'une solution aqueuse de carbonate alcalin à des températures de 20 à 95 °C, filtration, lavage, éventuellement façonnage de la masse de catalyseur, puis réduction par l'hydrogène à des températures de 200 à 300 °C, de préférence de 220 à 280 °C.

2. Procédé selon la revendication 1, caractérisé en ce que le catalyseur contient en outre de 0,25 à 15 % en poids, par rapport à sa masse totale, de $SiO_2$, de $MnO_2$, de $ZrO_2$, de $Al_2O_3$ ou de MgO isolément ou à l'état de combinaison de deux ou plusieurs de ces substances à l'état d'oxydes, d'hydroxydes ou d'oxydes hydratés.

3. Procédé selon la revendication 2, caractérisé en ce que le catalyseur contient $SiO_2$, $MnO_2$, $ZrO_2$, $Al_2O_3$ ou MgO en quantité de 1 à 8 et plus spécialement de 2 à 5 % du poids de la masse totale de catalyseur.

4. Procédé selon une ou plusieurs des revendications 1 à 3, caractérisé en ce que la réduction est effectuée en au moins trois stades opératoires avec une température croissant d'un stade à l'autre.

5. Procédé selon la revendication 4, caractérisé en ce que la réduction est effectuée au premier stade à une température de 220 à 250 °C, de préférence de 230 à 240 °C, au deuxième stade à une température de 245 à 260 °C, de préférence de 250 à 255 °C et au troisième stade à une température de 255 à 280 °C, de préférence de 260 à 270 °C.

6. Procédé selon une ou plusieurs des revendications 1 à 5, caractérisé en ce que l'hydrogénation est effectuée à des températures de 45 à 100 °C, de préférence de 50 à 80 °C.

7. Procédé selon une ou plusieurs des revendications 1 à 6, caractérisé en ce que l'hydrogénation est effectuée à une pression de 6 à 12, de préférence de 8 à 10 MPa.

8. Procédé selon une ou plusieurs des revendications 1 à 7, caractérisé en ce que les dialkylaminoacétonitriles sont mis en oeuvre à l'état de solution dans un solvant inerte.

9. Procédé selon la revendication 8, caractérisé en ce que la concentration du dialkylaminoacétonitrile dans la solution est de 5 à 50 % en poids, de préférence de 10 à 40 % en poids et plus spécialement de 15 à 30 % en poids.

10. Procédé selon une ou plusieurs des revendications 1 à 9, caractérisé en ce que l'hydrogénation est effectuée en présence d'ammoniac, avec mise en oeuvre de l'ammoniac en quantité de 1 à 20 mol, de préférence de 1,5 à 15 mol et plus spécialement de 2 à 10 mol par mole du dialkylaminoacétonitrile.